# EUROPEAN PATENT SPECIFICATION

(11) **EP 0 422 209 B1**
(45) Date of publication and mention of the grant of the patent: **15.03.1995**
(21) Application number: 90907948.5
(22) Date of filing: 25.04.1990
(51) Int. Cl.: A61L 27/00

(54) **IMPLANTS FOR LARGE VOLUMES OF CELLS ON POLYMERIC MATRICES**
IMPLANTATEN FÜR GROSSE MENGEN VON ZELLEN AUF POLYMERISCHE MATRIZEN
IMPLANTS POUR GRANDS VOLUMES DE CELLULES SUR DES MATRICES POLYMERES

(30) Priority: 25.04.1989 US 343158
(43) Date of publication of application: 17.04.1991
(73) Proprietor: CHILDREN'S MEDICAL CENTER CORPORATION, Boston Massachusetts 02115 (US); MASSACHUSETTS INSTITUTE OF TECHNOLOGY, Cambridge, MA 02139 (US)
(72) Inventor: Vacanti, Joseph P., Winchester, MA 01890 (US); Langer, Robert S., Newton, MA 02158 (US); Johnson, Lynt, Randolph, MA 02368 (US); Griffith-Cima, Linda, Lexington, MA 02173 (US)
(74) Representative: Bassett, Richard Simon
(86) International application number: US9002257
(87) International publication number: WO9012604

(56) References cited:
- WO-A-88/03785
- US-A- 4 637 931

## Description

### Background of the Invention

This invention generally relates to organ implantation and more specifically relates to a method for implanting large volumes of cells on polymeric matrices into a patient.

There are many diseases which cause significant dysfunction of the liver, ultimately causing hepatic failure. There are no artificial support systems for liver failure, so that, in the absence of a successful transplant, liver failure always results in the death of the patient. It has been estimated that 30,000 people die of hepatic failure every year in the United States, at a cost to society of $14 billion dollars annually. Some of these diseases include genetic defects that result in defects of protein metabolism, defects of amino acid metabolism, defects of carbohydrate metabolism, defects of pyrimidine and purine metabolism, defects of lipid metabolism, and defects of mineral metabolism. Another group of patients suffering from liver disease are those with alcohol induced liver disease. At this time, these patients have no options.

Over the last few years, organ implantation has become an increasingly important method for treating organ dysfunction. Unfortunately, despite the current success in transplantation of a variety of organs, especially the liver, many people die as a result of the critical shortage of donor organs. The only method for treating those patients for which transplantation is an option is to maintain them until a liver becomes available for transplantation.

Transplantation of the whole liver has become an increasingly successful surgical manipulation through the 1980's, largely through the efforts of Dr. Thomas Starzl. However, the technical complexity of the surgery, the enormous loss of blood, the stormy postoperative course, and the many unknowns of hepatic transplantation, have made it an expensive technology available only in major medical centers. It has become increasingly clear that because of donor scarcity, transplantation will never meet the needs of the patients who require it. Currently, approximately 1500 patients per year undergo hepatic transplantation. Even if that capacity were tripled, it would fall short of the 30,000 patients dying of end-stage liver disease.

The emergence of organ transplantation and the science of immunobiology has allowed replacement of the kidney, heart, liver, and other organs. However, as the ability to perform these complex operations has improved, the limitations of the technology have become more evident. The surgery is complex and usually associated with major blood loss. The preservation time is short and, therefore, results in major logistical problems in matching a distant donor with a recipient. For these reasons, the undertaking is expensive and labor intensive, requiring a major investment of resources available only in tertiary care facilities.

Selective cell transplantation of only those parenchymal elements necessary to replace lost function has been proposed as an alternative to whole or partial organ transplantation. This has several attractive features, including avoiding major surgery with its attendant blood loss, anesthetic difficulties, and complications. It replaces only those cells which supply the needed function and, therefore, problems with passenger leukocytes, antigen presenting cells, and other cell types which may promote the rejection process are avoided. Adding the techniques of cell culture provides another set of tools to aid in the transplantation process. The ability to expand cell numbers with proliferation of cells in culture, in theory, allows autotransplantation of one's own tissue.

Recently, a number of groups have engaged in research and development of various ways to grow cells in vitro for subsequent implantation, as well as to directly implant the cells in vivo. Most such efforts have met with only limited success due to problems with the cells failing to proliferate and function once implanted.

WO87/06120 by Marrow-Tech Incorporated describes successfully growing in vitro cells such as bone marrow cells on nylon meshes seeded with stromal cells. A.A.Demetriou,et al., Science 233,1190-1192 (1986) describes implantation and function of hepatocytes attached to collagen coated microcarrier beads injected into the peritoneal cavity. Others have directly implanted in vivo pancreatic tissue into diabetic patients. An earlier approach which was not successful in achieving long-term benefits was the transplantation of islet cells through injection of isolated clusters of islet cells into the portal circulation, with implantation in the vascular bed of the liver. More recent experimental methods have included encapsulation of pancreatic beta cells to prevent immune attack by the host and injection of fetal beta cells beneath the capsule of the kidney. Although there is evidence of short term function, long term results have been less satisfactory (D.E.R. Sutherland, Diabetologia 20, 161-185 (1981); D.E.R. Sutherland, Diabetologia 20,435-500 (1981)), and whole organ pancreatic transplantation has remained the preferred treatment.

WO-A-8803785 entitled "Chimeric Neomorphogenesis of Organs by Controlled Cellular Implantation Using Artificial Matrices" filed by Joseph P. Vacanti and Robert S. Langer discloses methods and matrices that allow cells of a variety of types to be proliferated in vitro prior to implantation in vivo and vascularization. The principal element of both the method and the matrices is that the three dimensional support structure provides sufficient spacing between seeded cells for adequate diffusion of nutrients and gas exchange from the surrounding media to occur in the absence of vascularization. This was developed in response to the problem that the inventors had observed empirically when "thick" structures were implanted, where the structures had inadequate interstitial spacing to allow free diffusion of nutrients and gases through the structure to maintain cell viability until vascular ingrowth had occurred.

This method utilizes structures having a diameter of greater than 300 microns, which are initially cultured *in vitro*, then implanted *in vivo*. As a result, a large mass including cells must be implanted into the patient using surgical procedures creating a wound which can then produce complications. Further, it requires implantation of a large volume of cells for the cells to proliferate and function.

It is therefore an object of the present invention to disclose a method and means for implanting large volumes of cells to form a variety of organs, especially endocrine organs including liver, pancreas and adrenal gland, which functionally resemble the naturally occurring organs.

It is a further object of the present invention to provide a method for implanting large volumes of cells on biodegradable, non-toxic matrices to form functional organ equivalents which is relatively non-invasive and traumatic as compared to conventional surgical procedures.

### Summary of the Invention

The present invention is a matrix sheet structure and use of a matrix structure whereby large volumes of cells having a desired function are attached to polymer scaffolding and transferred with minimal wounding and blood loss into a patient at a site appropriate for attachment, growth and function of the cells on the scaffolding, thereby producing a functional organ equivalent. One aspect of the invention provides the use of a matrix structure comprising a biocompatible material in a fibrous shape having interstitial spacing in the range of 100 to 200 »m and having viable cells attached thereto in the manufacture of a surgical implant for use in a method of surgery comprising implanting a plurality of matrices between folds of tissue having high surface area and vasculature adjacent the surface of the tissue.

A further aspect of the invention provides a matrix sheet structure comprising a biocompatible material in a fibrous shaping having interstitial spacing in the range 100 to 200 »m and having viable parenchymal cells attached thereto wherein the thickness of the matrix structure is in the range 200 »m to 2 mm. The method involves seeding cells onto a number of similar or different matrices, then implanting the matrices in vivo between tissues so that the implanted cells are provided with adequate nutrition and gas exchange, even in the absence of vascularization, but in cell quantities sufficiently large to provide the required function. The method is particularly well suited for growth of endocrine structures, including liver, pancreas, and adrenal gland, but can be used for growth and function of other types of tissue.

In the preferred embodiment, seeded polymer sheets are placed between folds of the mesentery. The vascular supply from the portal circulation supplies nutrients and normal metabolic factors to the implanted cells by diffusion until ingrowth of blood vessels following implantation provides for normal feedback mechanisms controlling the soluble products of the implanted cells. The preferred material for forming the matrix or support structure is a biodegradable artificial polymer, which is degraded by hydrolysis at a controlled rate and absorbed, alone or in combination with a non-degradable support structure. The degradable materials provide the maximum control of degradability, manageability, size and configuration. Further, materials such as angiogenesis factors can be incorporated into degradable matrices for use in preparing the implantation sites prior to, or at the time of, implanting the cells.

Initially growing the cells in culture allows manipulation and proliferation of the cells which may be beneficial following implantation of the matrix cell structures, but is not required if adequate cells for seeding can be obtained by biopsy.

Two hundred rat implantation studies using new tissue laminates of mesenteric leaves alternating with felt-like sheets of polymer-hepatocytes constructs have been conducted. Hepatocyte loading on polymers in culture has varied between 30 and 600 million cells per rat, averaging 60 to 100 million cells per rat. A 150 gram rat accepts 36 cm² of polymer material loaded with cells (eight sheets 1x3 cm x 2 mm thickness seeded to 500,000 cells/cm², yielding an implant 1x1x3 cm). Engraftment has been achieved in 96% of cases. Histological analysis from 5 days to ten months reveals neovascularization, histologically normal appearing nests and clusters of hepatocytes. Liver specific function has been documented *in situ* at 62 days using immunofluorescent staining for albumin, and partial replacement of function has been observed in the Gunn rat model of glucuronyl transferase deficiency.

### Brief Description of the Drawings

Figure 1A is a side saggital sectional view of an adult human showing the various elements of the upper and lower gastrointestinal tract, including the small intestines and the mesentery. Figure 1B is a frontal prospective view of the mesentery of the small intestine.

Figure 2 is a view of the veins of the circulatory system associated with the mesentery.

Figure 3A and 3B are diagrams of the method of the present invention showing implantation of polymer sheets seeded with cells being placed between folds of the mesentery; Figure 3A is a prospective view; Figure 3B is a cross-sectional view.

Figure 4 is a diagram of the chimeric structure resulting from the insertion of the polymer sheets, as shown in Figure 3, loosely approximated together to form a chimeric cell matrix structure according to the present invention.

Figure 5 is a freeze-fracture view of a cross-section of a sponge-like matrix having a thickness in the range 200 to 500 »m thick.

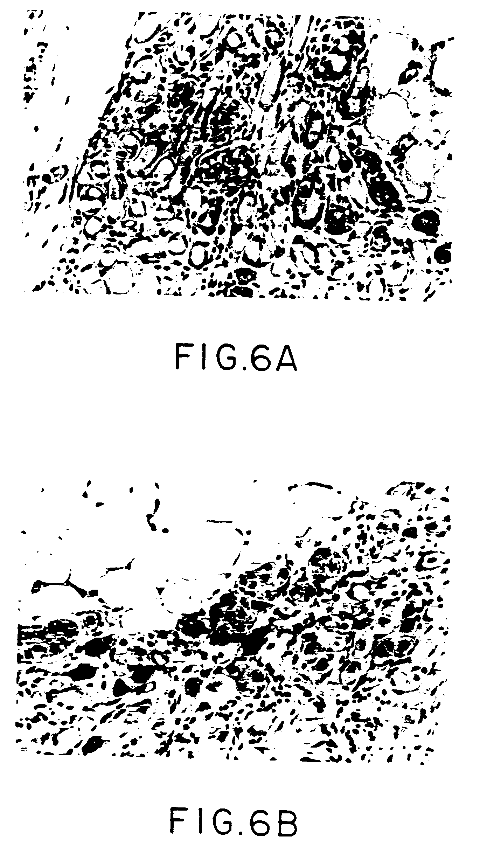

Figure 6A depicts a low power view of an implantation site. The right and left margins are the original mesenteric folds and the cell polymer construct can be seen between the leaves. The polymer material shows a giant cell foreign body reaction around each of the fibers. There is a modest inflammatory cell infiltrate in the interstices. Small nests and clusters of viable hepatocytes can be seen throughout the implant, and neovascularization is present throughout. Figure 6b is a higher power view documenting that the nests of hepatocytes clearly prefer the edges of the implant closest to the mesenteric fold and the associated blood supply. The hepatocytes are also not attached to the polymer fibers, indicating the preferential adhesivity that occurs to each other as well as to the matrix that they have laid down. There is an associated fibroblast reaction with collagen deposition.

Figure 7a is a photo of a polymeric matrix containing GHL implanted into the mesentery. Figure 7b is a photo of a polymeric matrix not containing GHL implanted into the mesentery.

### Detailed Description of the Invention

A method allowing large volumes of avascular tissue to be simultaneously implanted in a patient with minimal wounding is based on the discovery that multiple seeded polymeric matrices can be juxtaposed with certain tissues and undergo adequate exchange of nutrients and gases to grow and proliferate. These tissues, such as the mesentery and the omentum, have large surface areas and are highly vascular.

### Method and Site of Implantation of Cell-Matrix Constructs.

The peritoneum is an extensive serous membrane lining the abdominal cavity and many of the organs in the cavity. The "mesentery" is ordinarily used to refer to the mesentery of the small intestine, a double-layered fold of peritoneum suspending it from the posterior abdominal wall. The attached border of the mesentery is only about 15 cm in length, and runs from approximately the second lumbar vertebra downward and to the right, crossing part of the duodenum, the aorta, the inferior vena cava, and across towards the right sacroiliac joint. The free or unattached border, containing the jejunaileum, is frilled out like an accordion, attaining a length ranging from three to six meters. The distance from the free border to the attached border ranges from 15 to 22 cm in length.

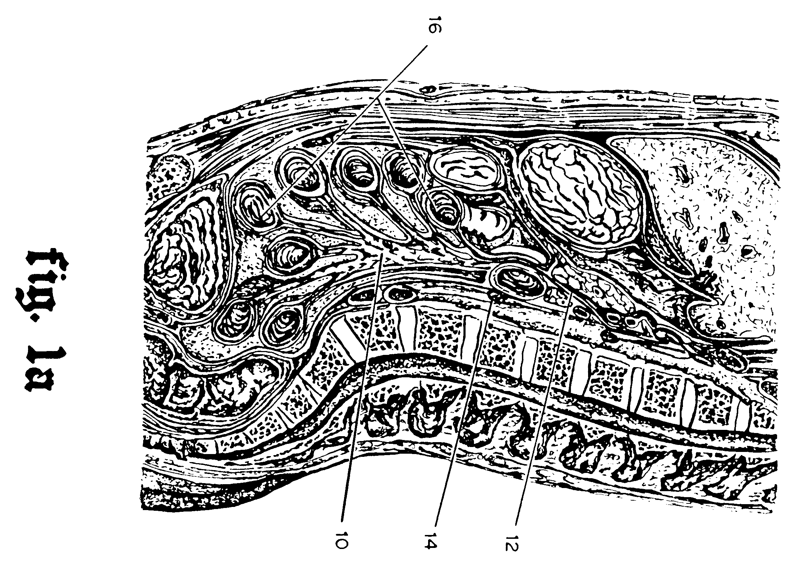

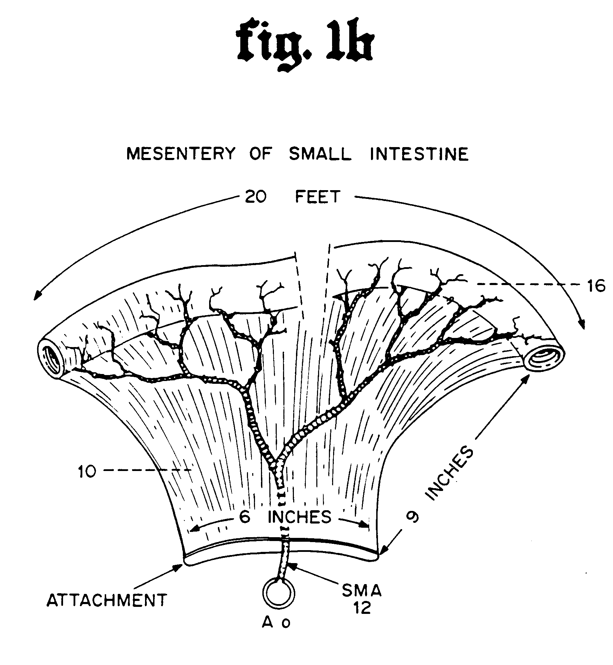

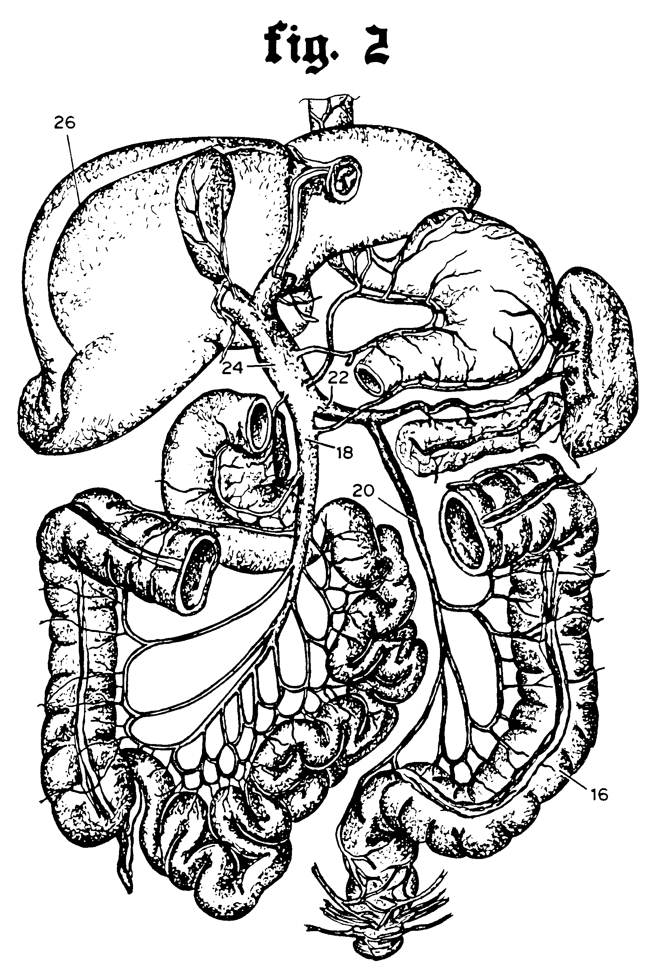

Between the two layers of peritoneum, on the two surfaces of the mesentery, are the superior mesenteric artery and its branches, the accompanying veins, lymphatics, lymph nodes, connective tissue, and varying amounts of adipose tissue. The mesentery is shown in cross section in Figures 1A and 1B. With reference to Figure 1A, the mesentery 10 is connected to the superior 12 and inferior 14 mesenteric arteries, supplying blood to the small intestine 16. With reference to Figure 1B, the mesentery 10 expands outwardly like a fan to the small intestine 16, the superior mesenteric artery 12 draining into the superior mesenteric vein. As shown in Figure 2, the inferior mesenteric vein 20 drains blood from the splenic 22, coronary and pyloric veins. The superior mesenteric vein 18 drains into the portal vein, leading to the liver 26. The vascular supply from the portal circulation supplies nutrients and normal metabolic factors to the implanted cells by diffusion until ingrowth of blood vessels following implantation provides for normal feedback mechanisms controlling the soluble products of the implanted cells.

The omentum is a double fold of peritoneum attached to the stomach and connecting it with some of the other organs, including the intestines. Other sections of the peritoneum and isolated tissues having similar characteristics can also be used in the method for implanting large volumes of cells.

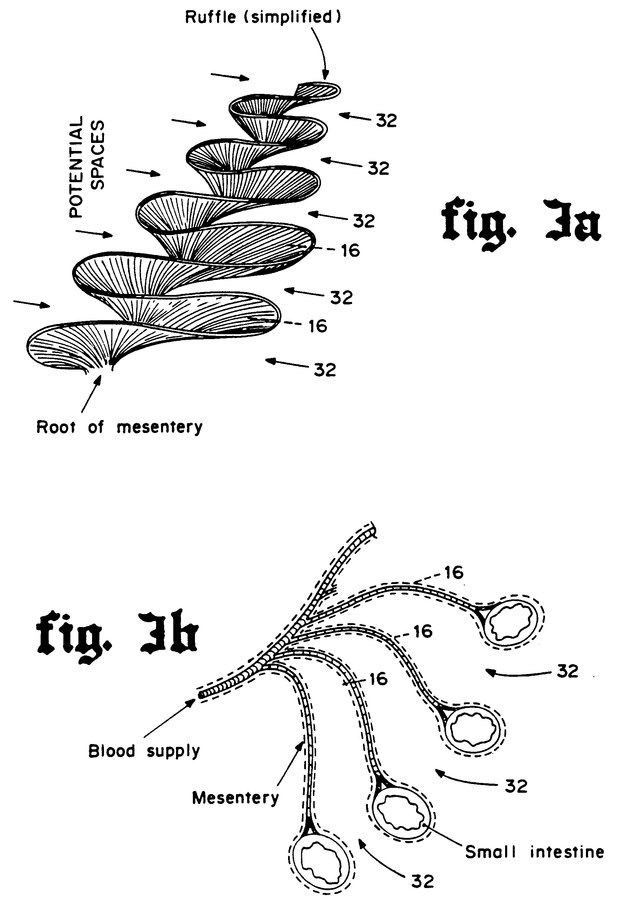

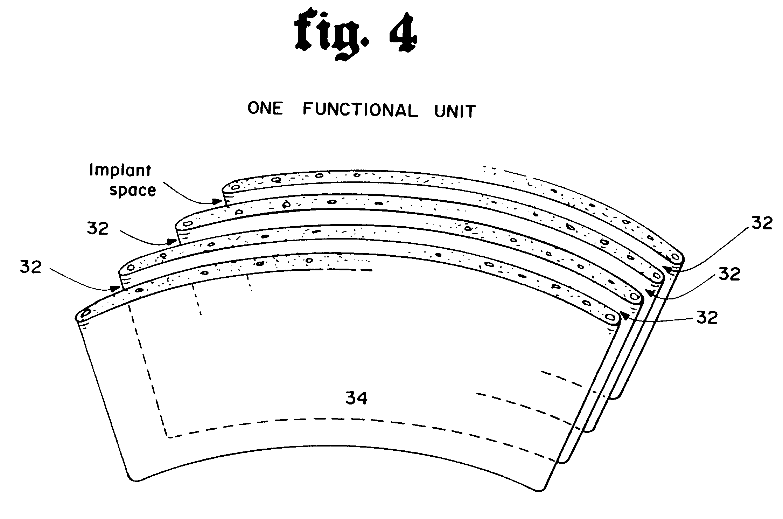

As shown schematically in Figures 3 and 4, chimeric cell-polymeric structures are formed by seeding biodegradable, biocompatible high surface area matrices with cells, derived from biopsy of the patient or a close relative or from cell culture, and implanting the seeded matrices between folds 32 of the mesentery 16. The folds 32 of the mesentery are approximated together to form a chimeric structure 34.

### Selection of Matrix Material.

The preferred material for forming the matrix or support structure is a biodegradable artificial polymer, for example, polylactic acid, polyglycolic acid, polyorthoester, polyanhydride, blends or copolymers thereof, which is degraded by hydrolysis at a controlled rate and absorbed, alone or in combination with non-degradable materials. The biodegradable materials provide the maximum control of degradability, manageability, size and configuration. However, other biocompatible polymeric materials, including collagen and non-biodegradable materials, can be used to form the structures.

Examples of non-degradable, or non-absorbable, materials include polypropylene, polyethylene terephthalate (Dacron polyester) and other polyesters, Teflon polytetrafluoroethylene; Teflon is a trade mark, ethylene vinyl acetate, nylon and stainless steel. Nylon is not preferred because it undergoes slow but constant hydration and degradation. Stainless steel filaments are difficult to fabricate. Both polypropylene and polyester are FDA approved as suture materials and as other implant components. They can be easily fabricated into monofilament, yarn or staple with varying fiber diameters. Both materials have very low orders of tissue reaction.

In some embodiments these materials are overlaid with a second material such as basement membrane components, agar, agarose, gelatin, gum arabic, collagens, fibronectin, laminin, glycosaminoglycans, mixtures thereof, and other materials having properties similar to biological matrix molecules known to those skilled in the art of cell culture to enhance cell attachment. These materials can also affect longevity and maintenance of several liver-specific functions, including production of albumin. Studies conducted using extracellular gel matrix (EHS) have shown that hepatocytes cultured on polymers coated with this material continue to transcribe at least two representative liver specific genes, albumin and α-1-inhibitor 3 at high rates. Cytoskeletal gene transcription under the same conditions is low. Both rates, as determined by nuclear run-on assays, resemble normal liver. Moreover, the abnormal patterns of mRNA abundance with respect to liver-specific and cytoskeletal genes are negated on EHS. Collagen is approved by the FDA for both suture and implant use. Some collagens also promote angiogenesis and fibrosis which may be advantageous in some instances.

Cellular differentiation and growth can be controlled by varying attachment site density. Attachment site density can be altered by selection or chemical modification of the polymeric support material, or by coating the substrate with a predetermined number of attachment molecules. For example, a high pH carbonate buffer can be used to adsorb purified extracellular matrix (ECM) molecules, such as laminin and fibronectin, onto polymer substrates. Hepatocytes attached to low densities of purified ECM (one to 50 ng/cm²) maintain a round morphology, high secretion rates for hepatocyte specific proteins (albumin, transferrin, fibrinogen), and low levels of DNA synthesis. Hepatocytes attached to high densities of purified ECM (500 to 2000 ng/cm²) spread extensively, have lower secretion rates for the hepatocyte specific proteins, and high levels of DNA synthesis. This control is largely independent of the ECM molecule used, although there is a small effect on differentiation depending on the specific ECM molecule used.

The major advantage of the biodegradable material is that it does not have to be removed once cell growth and formation of a functional mass has occurred. Another advantage of the biodegradable material is that compounds may be incorporated into the matrix for slow release during degradation of the matrix. For example, angiogenic compounds, nutrients, growth factors, inducers of differentiation or de-differentiation, products of secretion, immunomodulators, inhibitors of inflammation, regression factors, biologically active compounds which enhance or allow ingrowth of the lymphatic network or nerve fibers, and drugs can be incorporated into the matrix or provided in conjunction with the matrix, in solution or incorporated into a second biodegradable polymer matrix.

### Matrix Configuration.

In the preferred embodiment, multiple polymeric matrices formed of fibrous sheets are inserted into either a fresh bed or a prevascularized bed in the mesentery to create a structure having overall dimensions between a few microns and several centimeters. Matrices can be made in a variety of shapes, taking into consideration the requirements of adequate surface area for attachment of the number of cells required for implantation and formation of a function organ equivalent, and the requirements of adequate spacing between surfaces of attachment for nutrients and gases to diffuse into the interior of the matrices to each attached cell. The latter requirement can be met by providing many relatively thin matrices and implanting each sheet between folds of the mesentery, or by providing matrices having overall dimensions greater than the maximum diffusion distance for nutrients into an equal volume of cells, as described in WO-A-8803785 entitled "Chimeric Neomorphogenesis of Organs by Controlled Cellular Implantation Using Artificial Matrices" by Joseph P. Vacanti and Robert S. Langer.

In the preferred embodiment, the matrix is fibrous in nature. This can range from a non-woven felt-like mesh to a overlying or entangled single fibers to a sponge-like structure. In addition to the three major variables in the matrix components of composition, reinforcement (if any), and coating, other variables including fiber diameter, fiber density, knitted constructions, and fiber cross-over fusion or entanglement, must be considered. These matrices can be characterized *in vitro* on the basis of dry and wet compressibility, compressibility after absorbable component is removed (in the case of a mixture of absorbable and non-absorbable materials), breaking strength, suture pull-out strength, collagen distribution and microscopy. *In vivo*, matrices are assessed on the basis of attachment and viability of the cells is determined.

Fibrous materials are commercially available in the form of sutures and non-woven felt-like materials. Suitable matrices can also be formed using standard techniques known to those skilled in the art such as solvent casting, spin casting or extrusion, or similar methodology. A useful felt-like material can be obtained from Davis & Geck. These types of fabrics made from a variety of biodegrading polymers have been used in many clinical applications and display very good tissue and vascular ingrowth when thinned to approximately 1/3 to 1/2 mm.

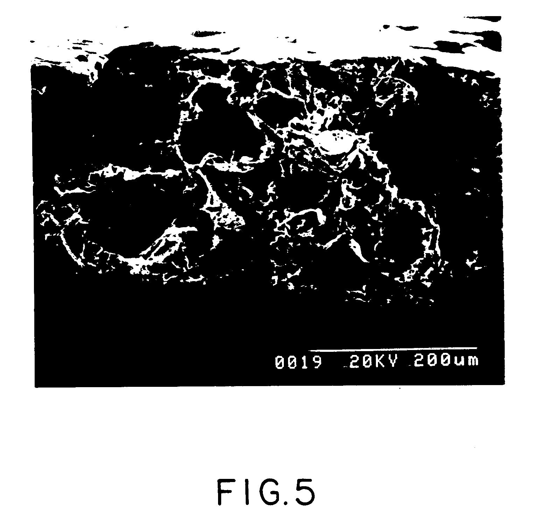

Sponge-like fibrous matrices have been made by solvent casting a polymer solution containing leachable particulates. For example, PLA/PLGA polymers have been cast containing NaCl particles sieved to the size range of 75 to 150 »m. The matrices were cast from a 10 wt% solution in methylene chloride into glass petri dishes, and the solvent was allowed to evaporate at room temperature under an atmosphere of methylene chloride. A 50/50 w/w blend of poly(DL) lactic acid, Mw 50,000 (Polysciences) and Dupont Medisorb 85:15 gave optimum results in terms of uniformity and porosity. These matrixes are 200 to 300 »m in thickness. A scanning electron micrograph of an example of this type of matrix is shown in Figure 5. The pore size for sponge-like structures, or interstitial spacing for fabrics, should be in the range of 100 to 200 »m for optimal ingrowth of vasculature.

A sponge-like matrix can also be made by dipping the Davis & Geck poly(glycolic acid) mesh into a polymer solution, such as poly(lactic acid) or a copolymer of poly(lactic acid) and poly(glycolic acid), where the polymer is dissolved in a solvent for the second polymer but not the poly(glycolic acid), such as methylene chloride. The second polymer greatly increases the compressive strength of the poly(glycolic acid) mesh and forms a "meniscus" between the fibers which harden into flat surfaces for cell attachment. In the preferred embodiment, the PGA fabric is dipped into a two to twenty weight percent PLA:GA 85:15 solution in methylene chloride, excess polymer solution is blotted away, and the matrix is air dried. This method is applicable to any biocompatible polymer or substrate which is not soluble in a second biocompatible, biodegradable polymer solution.

The surface area of the matrix or matrices to be implanted is determined based on the size of the recipient, as well as the type of cell(s) to be implanted. The average cell implant for a rat varies between 60 and 100 million cells per animal. This has been implanted using a surface area of approximately 36 cm² of polymer material. It is calculated that the raw available surface area of mesentery that could be used for implantation for a 70 kg adult human is 2.68 m². Modest cell application densities in the range of 700,000 cells per cm² could theoretically allow implantation of 10⁹ cells per adult human, 10% of the size of an adult human liver.

### Preparation of Implantation Site prior to Implantation of Cell-Matrix Construct.

In another embodiment of the invention, a matrix containing one or more of these biologically active compounds is implanted in the tissue prior to implantation of seeded matrices, to prepare the implantation site, for example, using angiogenic compounds to pre-vascularize the site.

### Selection and Attachment of Cells to the Matrix.

A variety of different cells can be seeded onto the matrix. In the preferred embodiment, endocrine cells such as hepatocytes, pancreatic cells or cells of the adrenal gland are proliferated on the matrices. Other cells, such as cells of the nervous system, including hypothalamus and pituitary cells, lymphoid cells, mesodermal cells, such as fibroblasts, endothelial cells, and lymphatic cells, splenic cells, and cells of the genitourinary system, for example, renal endocrine tissues, and sex related endocrine tissues, can also be implanted using this method.

With respect to the endocrine cells, where the cells are positioned on multiple matrices within the mesentery, the method locates these cells within the blood stream in close proximity to the blood supply the organs normally receive, between the portal and systemic systems. This exposes the cells to many of the factors present in the blood that aid in normal growth and proliferation.

Cells of one or more types can be selected and grown on the matrix. The matrix structure and the length of time and conditions under which the cells are cultured *in vitro* are determined on an individual basis for each type of cell by measuring cell attachment (only viable cells remain attached to the polymers), extent of proliferation, and percent successful engraftment. As discussed above, it is not necessary to culture cells *in vitro*, other than for purposes of attaching the cells to the matrix, prior to implantation if sufficient numbers of cells are available. Cells generally attach within a few hours. The most efficient technique for attaching cells to the mesh is to place a concentrated suspension of cells on the surface of the polymer, which is hydrophobic, and to allow the cell suspension to wick into the fabric over a period of about 30 min. The cells attach to the fibers mostly as individual cells but also in groups of two or three. Within the first twenty-four hours, the cells begin rearranging into clusters; at this point, some cells can be seen interacting a great deal with the fibers by wrapping all the way around an individual fiber. Within three days, the cells are almost completely organized into large clusters and groups of cells, and interact mostly with each other and not the fiber support.

Cells can be obtained by biopsy, surgical excision from a donor, or from established cell lines. Methods for dissociation of tissue are known but may need to be optimized for cell type and source. For example, hepatocytes are dissociated using enzymes such as collagenase, by mechanical disruption, and/or treatment with chemical agents such as ethylenediamine tetraacetic acid (EDTA) and tetraphenylboron.

In some cases it is advantageous to administer an immunosuppressant such as cyclosporine after implantation of the cell-matrix to increase viability of the implanted cells. This is essential in the case of xenografts, as has been demonstrated using implants of rat liver cells into rabbits, and beneficial in the case of homologous grafts, as has been demonstrated using hepatocyte implants into the enzymatically deficient Gunn rat.

Initially growing the cells in culture allows manipulation of the cells which may be beneficial following implantation of the matrix cell structure. Presently available technology allows the introduction of genes into the cells to make proteins which would otherwise be absent, such as those resulting from liver protein deficiencies and metabolic defects such as cystic fibrosis. Repression of gene expression may also be used to modify antigen expression on the cell surface, and thereby the immune response, so that cells are not recognized as foreign.

The present invention will be further understood by reference to the following non-limiting examples.

### Example 1: Isolation of Hepatocytes for Implantation on a Matrix in the Mesentery.

The method of Berry and Friend, J. Cell Biol. 43, 506-520 (1969), as adapted by Seglen, Methods in Cell Biology vol. 13, pp. 29-83 (San Diego, Ca, Academic 1976), was modified as follows to optimize the yield of hepatocytes, as well as viability.

Livers are perfused using a pump, autoclavable silastic tubing, a water bath and an air trap. The air trap, positioned to eliminate air bubbles in the perfusion buffers, is a very important component of the system. The liver can be perfused *in situ* or after removal from the body. If possible, *in situ* perfusion is preferred. *In situ*, the liver remains in the abdominal cavity through both steps of the perfusion and only following completion of the collagenase perfusion is surgically removed for dissociation into the primary cell culture. There is no recirculation of the collagenase perfusate.

Optimal results are obtained using Seglen's HEPES base initial buffer, pH 7.4, for the initial perfusion and the same buffer with 4.8 mmol/L CaCl₂ for the subsequent collagenase perfusion. The perfusion with the buffer clears blood and calcium from the liver. Five to six minutes for a rat liver is sufficient. The second step must continue long enough to effect good dissociation of the liver but not so long as to cause excessive damage to the cells. In general, five to ten minutes is optimal for the second step using rat liver, with some variation according to the age and weight of the donor and the activity of the collagenase. Optimal temperature of the water bath is 38 to 39 °C to produce a cannula output temperature of 35 to 36°C. These conditions consistently generate cell harvests of 500 to 700 x 10⁵ cells/gram of liver tissue with cell viability of 85 to 95%.

Hepatocytes were obtained from Fischer 34 and Gunn rats by collagenase perfusion. Cells were seeded onto non-woven filamentous sheets of polyglycolic acid 1 x 3 cm in size and 2 mm thick to 500,000 cells/cm². Recipient animals underwent laparotomy using sterile technique and sheets were placed between leaves of mesentery. Eight sheets were placed per animal and the leaves were approximated, creating a functional implant 1 x 1 x 3 cm.

Biopsy at day five post implantation revealed neovascularization, moderate inflammatory reaction, and the presence of viable hepatocytes.

This example demonstrates the successful implantation of large volumes of hepatocytes, cells which do not normally remain viable in the absence of a polymeric support, and which are difficult to proliferate in vivo to a number sufficient to form a functional organ equivalent, using multiple polymeric sheets placed into folds of the mesentery, with minimal trauma and blood loss.

### Example 2: Comparison of Absorbable Polymeric Matrix Materials and Coatings of Attachment Factors.

One of the key factors in the success of the transplant is the choice of the proper material for the construction of the scaffold or device which holds the cells for transplantation. The material should be biocompatible; it should also have properties that allow it to be fabricated into porous three dimensional devices with a high surface area/volume ratio to provide a significant surface area for cell attachment, and the resulting devices must have sufficient compressive strength to prevent collapse upon implantation. The material should be an adhesive substrate for the cells to be transplanted, and should ideally allow for retention of differentiated function by the cells and possibly for cell growth as well.

Synthetic polymers can be manufactured reproducibly and have good mechanical properties. Furthermore, use of degradable polymers should preclude long-term infections and foreign body reactions that would prevent integration of the transplanted cells into the proper tissue architecture.

From the perspective of biocompatibility, biodegradability, and ability to be processed, polyesters in the polylactide (PLA), and polylactide-co-glycolide (PLGA) family have many ideal features. The interaction parameters of interest are cell adhesion, longevity, and maintenance of differentiated function. Adhesion is desired because the hepatocytes are anchorage-dependent and because it is unlikely that non-adherent cells will stay localized to the site of the implant. It is also desired that cells remain attached and viable on the substrate; and that the attachment sites not degrade during the time span attachment is desired. A substratum that allows retention of function *in vitro* is the optimal substratum for functional retention *in vivo*. The functional viability of the cells may be altered if necessary by coating the polymer substratum with extracellular matrix proteins.

Substrates were made in the form of solvent cast films, and films of two different compositions were investigated. One set of films was made from poly(DL-lactide-co glycolide) with monomers in the ratio 85:15 lactide:glycolide (DuPont, Medisorb 85:15, weight ave Mw 40-100,000) and another set was made from a blend (50/50 w/w) of Medisorb 85:15 and poly(L-lactide) (Polysciences, Mw 50,000). Films were cast in 50 mm diameter glass petri dishes from a freshly made 15% polymer solution in methylene chloride (Mallinkrodt, analytical reagent grade); each film contained 0.4 gm polymer. The dishes containing the films were covered with petri dish covers and the solvent evaporated at room temperature for a minimum of 5 hr. The films were then placed under vacuum for 24-48 hr to remove residual solvent. The films were exposed to UV light for 90 min for purposes of sterilization and were stored desiccated until use. The films were prepared for culture by washing once with 5 ml phosphate buffered saline (PBS, pH 7.4) and then once with 5 ml complete culture medium. Control petri dishes (35 mm bacteriologic, Falcon #1008) were coated with Type I collagen (Vitrogen, Collagen Corp.) by adsorption from a 5 »g/ml solution of Vitrogen in 50 mM carbonate buffer (pH 9.4) for 16-20 hr at 4°C; the resulting surface concentration of collagen was 1 »g/cm².

Cells were isolated from 180-250 gm male Fisher rats using a modification of the two-step collagenase procedure of Seglen, P.O., Meth. Cell Biol., 13, 29-83 (1976). The liver was perfused in the retrograde direction first with Ca⁺⁺-free perfusion buffer (143 mM NaCl, 7 mM KCl, 10 mM HEPES, pH 7.4) for 6 min and then with the same perfusion buffer containing 5 mM CaCl₂ and 0.5 mg/ml collagenase (Worthington, Class II) until the liver became soft. Cells were dispersed in complete chemically defined serum-free culture medium (William's E with 10 ng/ml EGF (Collaborative Research), 20 mU/ml insulin (Gibco), 5 nM dexamethasone (Sigma), 20 mM pyruvate (Gibco), and 100 U/ml penicillin/streptomycin (Gibco)/(McGowan); cell viability following dispersion was 80-90% as determined by trypan blue exclusion. Dead cells and debris were removed by centrifugation in an iso-density Percoll solution (Kreamer, B.L., et al., In Vitro Cell. Dev. Biol., 22(4), 201-207 (1986)) and the resulting pellet was washed three times with complete medium prior to planting the cells. Viability at plating was 88-98%.

For routine culture, cells were plated at a concentration of 30,000 viable cells/cm² culture surface area (300,000 cells/dish for 35 mm control dishes, 600,000 cells dish for 50 mm polymer films; 150,000 cells/ml). Following an attachment period of 2-4 hr (maximum attachment to all substrates occurred within 90 min), the medium was changed to remove unattached cells and then cells were maintained in serum-free medium with daily medium changes.

Cell attachment to substrates was measured by direct counting or by determining relative protein content. For direct counting, cells were removed from the substrate using 0.05% trypsin/EDTA (Gibco). Cells plated on collagen-coated substrates required prolonged (30-45 min) treatment with trypsin for adequate cell dispersion, and plates were inspected visually before counting to ensure all cells had been removed from the substrate. For determining cell number on a large number of plates simultaneously, quantitative binding and extraction of the dye flavianic acid (NYS, Sigma)) was used (Skehan, P. and S.J. Friedman, In Vitro Cell. Devel. Biol., 21(5), 288-290 (1985)). The assay was calibrated for cell number by measuring the response of cells plated at two different concentrations (300,000 and 600,000 cells/plate) and counting identical plates seeded at the same concentrations; each point was measured in triplicate.

The rate of cell attachment was measured by seeding replicate plates at 30,000 cells/cm² surface area. At each time point, three identical plates were sacrificed for measurement of cell number by the NYS dye-binding assay.

For determining effects of the presence of extracellular DNA from dead cells on cell attachment at high cell concentrations (500,000 cells/cm²), deoxyribonuclease I (United States Biochemical Corp.) was added to the attachment medium at concentrations 0, 10, 100, and 1000 U/ml. Cells were plated on the standard vitrogen-coated 35 mm polystyrene dishes and attachment was determined on duplicate plates.

Albumin secretion into the culture medium was quantified by a sandwich ELISA technique (Schwerer, B., et al., Clin. Chim. Acta, 163, 237-244 (1987)) using antibodies specific for rat albumin (Organon-Teknika-Capell). DNA synthesis was measured biochemically by the incorporation of 1 »Ci/ml ³H-thymidine over 20 hr. Total DNA content and radioactivity were determined according to the method of McGowan, J.A. and N.L.R. Bucher, In Vitro, 19(3), 159-166 (1983).

Cell cultures were fixed in Karnovsky's fixative at 37°C for one hr, washed with 0.1 M cacodylate buffer (pH 7.4), postfixed for 1 hr in 1% osmium tetroxide, dehydrated in a graduated series of ethanol/water solutions, and dried in a critical point drier (Ladd) with supercritical CO₂. The samples were then sputter-coated with gold and observed in a Hitachi scanning electron microscope.

In one set of experiments, the kinetics of cell attachment to substrates were studied at cell surface concentrations of 30,000 cells/cm² and below to minimize the role of cell-cell interactions. (Note that there are two relevant cell concentrations: a two dimensional surface concentration and a volumetric concentration. The two-dimensional concentration is relevant because the cells settle to the surface quite quickly regardless of the total volume, and the surface concentration reflects the degree to which cells may interact at the attachment interface and the relative competition for sites; i.e., for cells 20-25 »m in diameter, total surface coverage with spheres would correspond to 160,000-250,000 cells/cm², so 30,000 cells/cm² would represent less than 20% surface coverage. The volume concentration is important because attachment may be inhibited by cell-secreted proteins.) The rate of attachment to both types of polymer films is similar and maximum attachment is achieved in 60 min, while maximum attachment to the collagen-coated control dishes required about 120 min. Hepatocytes were observed to attach primarily as single cells, and no difference in the kinetics of attachment to collagen-coated dishes was observed at a four-fold lower cell concentration (7500 cells/cm²).

At cell concentrations above 100,000 cells/cm², cell-cell interactions significantly affect the number of cells attaching to the substrate and the pattern of hepatocyte attachment is quite different for the polymer substrates and controls. At high cell concentrations, the cells were observed to form aggregates of 2-10 cells, and while these aggregates could be seen to attach to the collagen-coated substrates, they did not appear to interact with the polymer films. The formation of cell aggregates was not affected by the presence of DNAase (10-1000 U/ml), as would be expected if the leakage of DNA from dead cells was causing nonspecific aggregation. Even though a much greater number of cells attached to the collagen-coated substrates than to the polymer films at high cell concentrations, cell-cell interactions also influenced the pattern of attachment at concentrations of cells above 200,000/cm²; during the washing step, patches of cells would come up from the surface, leaving the surface bare in spots. The distribution of cells on the surface can be easily visualized during the NYS assay, and such patchiness was not observed at cell concentrations of 200,000 cells/cm² and lower.

The polymer substrates used for culture are opaque so scanning electron microscopy can be used to observe the morphology of the cells. The cells maintained on the collagen-coated polystyrene dishes were in general highly spread and flat; surface microvilli were observed predominantly in the center of the cells. In contrast, the morphology of cells on the polymer substrates was heterogeneous; both highly rounded and spread cells were observed. On the polymer films, both rounded and spread cells had numerous surface microvilli.

The growth pattern of hepatocytes maintained on polymer films cast from the blend of PLA and PLGA is similar to that of hepatocytes maintained on the collagen control substrates and the DNA synthesis rates are equivalent. Attrition of cells begins after three days in culture.

The films cast from pure PLGA 85:15 did not prove to be good substrates for cell longevity at the cell concentration investigated (20,000 cells/cm²). After 3-4 days, the cells detached from the substrate in large wisps or cords. Similar detachment of hepatocytes from the substrate when cells are cultured on fibronectin-coated polystyrene dishes (10 »g/cm² 10,000-30,000 cells/cm²) has been observed. Such detachment of cell sheets or cords from the substratum has also been observed in other systems and has been attributed to cell-cell tensions that overcome the cell-substratum tension.

The retention of hepatocellular function on polymer blend films was assessed by measuring the rate of albumin secretion. The rate of albumin secretion (»g/10⁶ cells/day) by cells maintained on the polymer blend films increased almost twofold over the five days in culture; in contrast, albumin secretion for cells on the collagen control dishes decreased over 60%. The decline in albumin synthesis for cells maintained on collagen was the same for collagen surface concentrations of 1 and 10 »g/cm². The secretion rate of albumin by cells on the polymer blend films is in the range of the reported *in vivo* rate for rats [17.3-19.4 mg/gm liver/24 hr (Peters, T.Jr., and J.C. Peters, J. Biol. Chem., 247(12), 3858-3863 (1972)) which corresponds to 144-162 »g/10⁶ cells/24 hr based on 120 x 10⁶ hepatocytes/gm liver.

The maximum cell concentration that could be accommodated on the polymer surface was 50,000 cells/cm², and the number of cells attached to the substrate declined if the number of cells plated at the surface exceeded 100,000 cells/cm². While the maximum in cell attachment of 50,000 cells/cm² may be a sufficient cell number in implant situations where cell growth is desired, higher cell attachment densities may be required if extensive cell-cell contact is necessary for retention of function by the implanted cells or if more efficient use of the polymer surface area is needed. Because a significantly greater number of cells attached to the collagen control, coating the polymer substrates with ng-»g /cm² amounts of extracellular matrix proteins such as collagen (or laminin or fibronectin) may be used to enhance cell adhesion for transplant matrices if high cell surface densities are required.

In order to retain hepatospecific function *in vivo*, it is expected that the cells will have to be implanted in a highly functional state, and that best way to ensure this is using a substratum for attachment which is also optimal for allowing retention of cell function. Films made from a blend of PLGA 85:15 and PLLA appear to allow excellent retention of one hepatocellular function (albumin secretion), although the mechanism of this retention is not clear because there is no biospecific interaction between the polymer and the cells. One possible mechanism of the observed retention of differentiated function is through modulation of cell shape; control of gene expression in many cell types has been demonstrated to be cell shape-dependent.

### Example 3: Prevascularization of a site for subsequent Implantation of a Cell Seeded Matrix.

There is a large loss of hepatocyte viability in the first 24 to 48 hrs after implantation. This loss occurs largely independent of the cell density that is used for implantation. However, the denser the application the large the number of viable cells that survive engraftment. Prevascularization appears to help by increasing the number of engrafted cells. Figure 6a depicts a low power view of an implantation site. The right and left margins are the original mesenteric folds and the cell polymer construct can be seen between the leaves. The polymer material shows a giant cell foreign body reaction around each of the fibers. There is a modest inflammatory cell infiltrate in the interstices. Small nests and clusters of viable hepatocytes can be seen throughout the implant, and neovascularization is present throughout. Figure 6b is a higher power view documenting that the nests of hepatocytes clearly prefer the edges of the implant closest to the mesenteric fold and the associated blood supply. The hepatocytes are also not attached to the polymer fibers, indicating the preferential adhesivity that occurs to each other as well as to the matrix that they have laid down. There is an associated fibroblast reaction with collagen deposition.

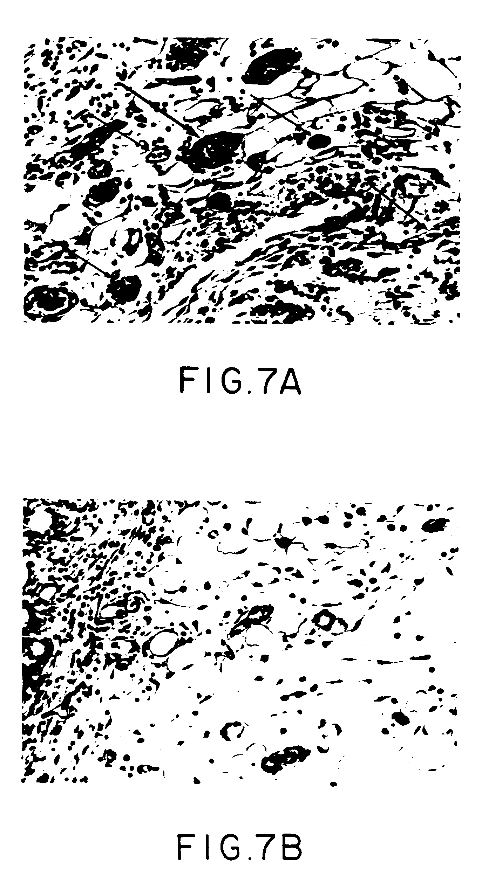

Prevascularization was accomplished by implantation of polymer loaded with the tripeptide glycine-histidine-lysine (GHL), a known potent angiogenesis agent as well as an hepatotrophic agent. Figure 7a is a photo of a polymeric matrix containing GHL implanted into the mesentery. Figure 7b is a photo of a polymeric matrix not containing GHL implanted into the mesentery. The matrices did not include cells. The polymer is in the upper left hand corners. The arrows in Figure 7a denote the large, dense vascular network that arose over the five-day implantation period of the GHL containing polymeric matrix.

Modifications and variations of the present invention, a method for implanting large volumes of functional cells on polymeric matrices in vivo, and the product thereof, will be obvious to those skilled in the art from the foregoing detailed description of the invention.

## Claims

1. The use of a matrix structure comprising a biocompatible material in a fibrous shape having interstitial spacing in the range of 100 to 200 »m and having viable parenchymal cells attached thereto in the manufacture of a surgical implant for use in a method of surgery comprising implanting a plurality of matrices between folds of tissue having high surface area and vasculature adjacent the surface of the tissue.

2. The use according to Claim 1 wherein the tissue is the mesentery, the omentum or the peritoneum.

3. The use according to Claim 1 or 2 wherein the material is a biodegradable polymer selected from the group consisting of polylactic acid, polyglycolic acid, polyorthoester, polyanhydride, collagen, and copolymers, blends and combinations thereof.

4. The use according to Claim 1 or 2 wherein the material is a non-degradable, or non-absorbable, material selected from the group consisting of polypropylene, polyethylene terephthalate and other polyesters, polytetrafluoroethylene, ethylene vinyl acetate, nylon, stainless steel, and combinations thereof.

5. The use according to any one of Claims 1 to 4 wherein the matrix material is coated with an attachment factor selected from the group consisting of basement membrane components, agar, agarose, gelatin, gum arabic, collagens, fibronectin, laminin, hyaluronic acid, glycosaminoglycans, attachment peptides, and mixtures thereof.

6. The use according to any one of Claims 1 to 5 wherein the matrix further comprises a compound selected from the group consisting of angiogenic compounds, nutrients, growth factors, inducers of differentiation or de-differentiation, products of secretion, immunomodulators, inhibitors of inflammation, regression factors, biologically active compounds which enhance or allow ingrowth of the lymphatic network or nerve fibres, and mixtures thereof.

7. The use according to any one of Claims 1 to 6 wherein the cells are selected from the group consisting of hepatocytes, pancreatic cells, adrenal cells, lymphoid cells, cells of the nervous system, fibroblasts, endothelial cells, lymphatic cells, splenic cells, and cells of the genitourinary system.

8. The use according to any one of Claims 1 to 7 wherein the matrix structure has a fibrous mesh-like shape.

9. The use according to any one of Claims 1 to 7 wherein the matrix structure has a sponge-like shape.

10. The use according to any one of Claims 1 to 7 wherein the matrix structure is a sheet.

11. The use according to Claim 10 wherein the thickness of the sheet is in the range 200 »m to 2 mm.

12. The use according to any one of Claims 1 to 3 or any of Claims 5 to 11 when not dependent on Claim 4 formed by coating a biocompatible fibrous mesh with a second biocompatible, biodegradable polymer solution, wherein the fibrous mesh material is not soluble in the second polymer solution.

13. The use according to Claim 12 wherein the fibrous mesh is formed of poly(glycolic acid) and the second polymer is selected from the group of poly(lactic acid) and poly(lactic acid-glycolic acid) copolymers.

14. The use according to Claim 12 or 13 wherein the second polymer forms flat surfaces between the fibers forming the fibrous mesh.

15. A matrix sheet structure comprising a biocompatible material in a fibrous shape having interstitial spacing in the range of 100 to 200 »m and having viable parenchymal cells attached thereto wherein the thickness of the matrix structure is in the range of 200 »m to 2 mm.

16. A matrix sheet structure according to Claim 15 wherein the thickness of the matrix structure is in the range of 200 to 300 »m.

17. A matrix sheet structure according to Claim 15 wherein the material is a biodegradable polymer selected from the group consisting of polylactic acid, polyglycolic acid, polyorthoester, polyanhydride, collagen, and copolymers, blends and combinations thereof.

18. A matrix sheet structure according to Claim 15 wherein the material is a non-degradable, or non-absorbable material selected from the group consisting of polypropylene, polyethylene terephthalate and other polyesters, polytetrafluoroethylene, ethylene vinyl acetate, nylon, stainless steel, and combinations thereof.

19. A matrix sheet structure according to any one of Claims 15 to 18 wherein the matrix material is coated with an attachment factor selected from the group consisting of basement membrane components, agar, agarose, gelatin, gum arabic, collagens, fibronectin, laminin, hyaluronic acid, glycosaminoglycans, attachment peptides, and mixtures thereof.

20. A matrix sheet structure according to any one of Claims 15 to 19 wherein the matrix further comprises a compound selected from the group consisting of angiogenic compounds, nutrients, growth factors, inducers of differentiation or de-differentiation, products of secretion, immunomodulators, inhibitors of inflammation, regression factors, biologically active compounds which enhance or allow ingrowth of the lymphatic network or nerve fibres, and mixtures thereof.

21. A matrix sheet structure according to any one of Claims 15 to 20 wherein the cells are selected from the group consisting of hepatocytes, pancreatic cells, adrenal cells, lymphoid cells, cells of the nervous system, fibroblasts, endothelial cells, splenic cells, and cells of the genitourinary system.

22. A matrix sheet structure according to any one of Claims 15 to 17 or any of Claims 19 to 21 when not dependent on Claim 18 formed by coating a biocompatible fibrous mesh with a second biocompatible, biodegradable polymer solution, wherein the fibrous mesh material is not soluble in the second polymer solution.

23. A matrix sheet structure according to Claim 22 wherein the fibrous mesh is formed of poly(glycolic acid) and the second polymer is selected from the group of poly(lactic acid) and poly(lactic acid-glycolic acid) copolymers.

24. A matrix sheet structure according to Claim 22 or 23 wherein the second polymer forms flat surfaces between the fibers forming the fibrous mesh.

## Patentansprüche

1. Verwendung einer Matrixstruktur, die ein biokompatibles Material in einer faserigen Form mit interstitiellen Abständen im Bereich von 100 bis 200 »m und mit daran angehefteten lebensfähigen parenchymalen Zellen umfaßt, zur Herstellung eines chirurgischen Implantats zur Verwendung in einem chirurgischen Verfahren, das die Implantation einer Vielzahl von Matrizen zwischen Gewebefalten mit großer Oberfläche und Vaskularisation nahe der Oberfläche des Gewebes umfaßt.

2. Verwendung nach Anspruch 1, wobei das Gewebe das Mesenterium, das Omentum oder das Peritoneum ist.

3. Verwendung nach Anspruch 1 oder 2, wobei das Material ein biologisch abbaubares Polymer ist, ausgewählt aus der Gruppe Polymilchsäure, Polyglykolsäure, Polyorthoester, Polyanhydrid, Kollagen und Copolymere, Mischungen und Kombinationen daraus.

4. Verwendung nach Anspruch 1 oder 2, wobei das Material ein nicht-abbaubares oder nicht-absorbierbares Material, ausgewählt aus der Gruppe Polypropylen, Polyethylenterephthalat und andere Polyester, Polytetrafluorethylen, Ethylenvinylacetat, Nylon, rostfreier Stahl und Kombinationen daraus, ist.

5. Verwendung nach einem der Ansprüche 1 bis 4, wobei das Matrixmaterial mit einem Anheftungsfaktor, ausgewählt aus der Gruppe Grundmembrankompopenten, Agar, Agarose, Gelatine, Gummi arabicum, Kollagene, Fibronektin, Laminin, Hyaluronsäure, Glycosaminoglycane, Anheftungspeptide und Mischungen daraus, beschichtet ist.

6. Verwendung nach einem der Ansprüche 1 bis 5, wobei die Matrix desweiteren eine Verbindung, ausgewählt aus der Gruppe angiogene Verbindungen, Nährstoffe, Wachstumsfaktoren, Induktoren einer Differenzierung oder Dedifferenzierung, Sekretionsprodukte, Immunmodulatoren, Entzündungshemmer, Regressionsfaktoren, biologisch aktive Verbindungen, die das Einwachsen des lymphatischen Netzwerkes oder der Nervenfasern erhöhen oder ermöglichen, und Mischungen daraus, enthält.

7. Verwendung nach einem der Ansprüche 1 bis 6, wobei die Zellen aus der Gruppe Hepatozyten, Pankreaszellen, Zellen der Nebenniere, lymphoide Zellen, Zellen des Nervensystems, Fibroblasten, Endothelzellen, lymphatische Zellen, Milzzellen und Zellen des Geschlechtssystems, ausgewählt sind.

8. Verwendung nach einem der Ansprüche 1 bis 7, wobei die Matrixstruktur eine faserige netzartige Form aufweist.

9. Verwendung nach einem der Ansprüche 1 bis 7, wobei die Matrixstruktur eine schwammartige Form aufweist.

10. Verwendung nach einem der Ansprüche 1 bis 7, wobei die Matrixstruktur die Form einer Lage besitzt.

11. Verwendung nach Anspruch 10, wobei die Dicke der Lage im Bereich von 200 »m bis 2 mm liegt.

12. Verwendung nach einem der Ansprüche 1 bis 3 oder einem der Ansprüche 5 bis 11, bei Nichtabhängigkeit von Anspruch 4, gebildet durch Beschichten eines biokompatiblen faserigen Netzes mit einer zweiten biokompatiblen, biologisch abbaubaren Polymerlösung, wobei das faserige Netzmaterial in der zweiten Polymerlösung nicht löslich ist.

13. Verwendung nach Anspruch 12, wobei das faserige Netz aus Poly(glykolsäure) gebildet ist und das zweite Polymer aus der Gruppe aus Poly(milchsäure) und Poly(milchsäure-Glykolsäure)-Copolymere, ausgewählt ist.

14. Die Verwendung nach Anspruch 12 oder 13, wobei das zweite Polymer zwischen den das faserige Netz bildenden Fasern flache Oberflächen ausbildet.

15. Matrixlagenstruktur, die ein biokompatibles Material in einer faserigen Form mit interstitiellen Abständen im Bereich von 100 bis 200 »m und mit daran angehefteten lebensfähigen parenchymalen Zellen umfaßt, wobei die Dicke der Matrixstruktur im Bereich von 200 »m bis 2 mm liegt.

16. Matrixlagenstruktur nach Anspruch 15, wobei die Dicke der Matrixstruktur im Bereich von 200 bis 300 »m liegt.

17. Matrixlagenstruktur nach Anspruch 15, wobei das Material ein biologisch abbaubares Polymer ist, ausgewählt aus der Gruppe Polymilchsäure, Polyglykolsäure, Polyortnoester, Polyanhydrid, Kollagen und Copolymere, Mischungen und Kombinationen daraus.

18. Matrixlagenstruktur nach Anspruch 15, wobei das Material ein nicht-abbaubares oder nicht-absorbierbares Material, ausgewählt aus der Gruppe Polypropylen, Polyethylenterephthalat und andere Polyester, Polytetra-fluorethylen, Ethylenvinylacetat, Nylon, rostfreier Stahl und Kombinationen daraus, ist.

19. Matrixlagenstruktur nach einem der Ansprüche 15 bis 18, wobei das Matrixmaterial mit einem Anheftungsfaktor, ausgewählt aus der Gruppe Grundmembrankomponenten, Agar, Agarose, Gelatine, Gummi arabicum, Kollagene, Fibronektin, Laminin, Hyaluronsäure, Glycosaminoglycane, Anheftungspeptide und Mischungen daraus, beschichtet ist.

20. Matrixlagenstruktur nach einem der Ansprüche 15 bis 19, wobei die Matrix desweiteren eine Verbindung, ausgewählt aus der Gruppe angiogene Verbindungen, Nährstoffe, Wachstumsfaktoren, Induktoren einer Differenzierung oder Dedifferenzierung, Sekretionsprodukte, Immunmodulatoren, Entzündungshemmer, Regressionsfaktoren, biologisch aktive Verbindungen, die das Einwachsen des lymphatischen Netzwerkes oder der Nervenfasern erhöhen oder ermöglichen, und Mischungen daraus, enthält.

21. Matrixlagenstruktur nach einem der Ansprüche 15 bis 20, wobei die Zellen aus der Gruppe Hepatozyten, Pankreaszellen, Zellen der Nebenniere, lymphoide Zellen, Zellen des Nervensystems, Fibroblasten, Endothelzellen, lymphatische Zellen, Milzsellen und Zellen des Geschlechtssystems ausgewählt sind.

22. Matrixlagenstruktur nach einem der Ansprüche 15 bis 17 oder einem der Ansprüche 19 bis 21 bei Nichtabhängigkeit von Anspruch 18, gebildet durch Beschichten eines biokompatiblen faserige Netzes mit einer zweiten biokompatiblen, biologisch abbaubaren Polymerlösung, wobei das faserige Netzmaterial in der zweiten Polymerlösung nicht löslich ist.

23. Matrixlagenstruktur nach Anspruch 22, wobei das faserige Netz aus Poly(glykolsäure) gebildet ist und das zweite Polymer aus der Gruppe Poly(milchsäure) und Poly(milchsäure-Glykolsäure)-Copolymere, ausgewählt ist.

24. Matrixlagenstruktur nach Anspruch 22 oder 23, wobei das zweite Polymer zwischen den die das faserige Netz bildenden Fasern flache Oberflächen ausbildet.

## Revendications

1. Utilisation d'une structure de matrice comprenant une matière biocompatible sous forme fibreuse ayant un espacement interstitiel dans la gamme de 100 à 200 »m et ayant des cellules parenchymateuses viables attachées à celle-ci, dans la fabrication d'un implant chirurgical pour l'utilisation dans un procédé de chirurgie comprenant l'implantation de plusieurs matrices entre des plis de tissu, ayant une aire superficielle élevée et un système vasculaire adjacent à la surface du tissu.

2. Utilisation selon la revendication 1, dans laquelle le tissu est le mésentère, l'omentum ou le péritoine.

3. Utilisation selon la revendication 1 ou 2, dans laquelle la matière est un polymère biodégradable choisi dans le groupe constitué d'un acide polylactique, d'un acide polyglycolique, d'un polyorthoester, d'un polyanhydride, du collagène et des copolymères, mélanges et combinaisons de ceux-ci.

4. Utilisation selon la revendication 1 ou 2, dans laquelle la matière est une matière non dégradable, ou non absorbable, choisie dans le groupe constitué du polypropylène, du polyéthylène téréphtalate et autres polyesters, du polytétrafluoroéthylène, de l'éthylèneacétate de vinyle, du nylon, de l'acier inoxydable et des combinaisons de ceux-ci.

5. Utilisation selon l'une quelconque des revendications 1 à 4, dans laquelle la matière de la matrice est revêtue avec un facteur d'attachement choisi dans le groupe constitué des constituants de membrane basilaire, de la gélose, de l'agarose, de la gélatine, de la gomme arabique, des collagènes, de la fibronectine, de la laminine, de l'acide hyaluronique, des glycosaminoglucanes, des peptides d'attachement et des mélanges de ceux-ci.

6. Utilisation selon l'une quelconque des revendications 1 à 5, dans laquelle la matrice comprend en outre un composé choisi dans le groupe constitué des composés angiogéniques, des substances nutritives, des facteurs de croissance, des inducteurs de différentiation ou de dé-différentiation, des produits de sécrétion, des immunomodulateurs, des inhibiteurs d'inflammation, des facteurs de régression, des composés biologiquement actifs qui accroissent ou permettent le développement interne du réseau lymphatique ou des fibres nerveuses, et des mélanges de ceux-ci.

7. Utilisation selon l'une quelconque des revendications 1 à 6, dans laquelle les cellules sont choisies dans le groupe constitué des hépatocytes, des cellules pancréatiques, des cellules surrénales, des cellules lymphoïdes, des cellules du système nerveux, des fibroblastes, des cellules endothéliales, des cellules lymphatiques, des cellules spléniques et des cellules du système génito-urinaire.

8. Utilisation selon l'une quelconque des revendications 1 à 7, dans laquelle la structure de la matrice a une forme semblable à un treillis fibreux.

9. Utilisation selon l'une quelconque des revendications 1 à 7, dans laquelle la structure de la matrice a une forme semblable à une éponge.

10. Utilisation selon l'une quelconque des revendications 1 à 7, dans laquelle la structure de la matrice est une feuille.

11. Utilisation selon la revendication 10, dans laquelle l'épaisseur de la feuille est dans la gamme de 200 »m à 2 mm.

12. Utilisation selon l'une quelconque des revendications 1 à 3 ou l'une quelconque des revendications 5 à 11 lorsqu'elles ne dépendent pas de la revendication 4, formée en revêtant un treillis fibreux biocompatible avec une seconde solution de polymère biocompatible, biodégradable, dans laquelle la matière du treillis fibreux n'est pas soluble dans la seconde solution de polymère.

13. Utilisation selon la revendication 12, dans laquelle le treillis fibreux est formé d'un acide polyglycolique et le second polymère est choisi dans le groupe d'un acide polylactique et des copolymères poly(acide lactique-acide glycolique).

14. Utilisation selon la revendication 12 ou 13, dans laquelle le second polymère forme des surfaces plates entre les fibres formant le treillis fibreux.

15. Structure de feuille de matrice comprenant une matière biocompatible sous forme fibreuse ayant un espacement interstitiel dans la gamme de 100 à 200 »m et ayant des cellules parenchymateuses viables attachées à celle-ci, dans laquelle l'épaisseur de la structure de la matrice est dans la gamme de 200 »m à 2 mm.

16. Structure de feuille de matrice selon la revendication 15, dans laquelle l'épaisseur de la structure de matrice est dans la gamme de 200 à 300 »m.

17. Structure de feuille de matrice selon la revendication 15, dans laquelle la matière est un polymère biodégradable choisi dans le groupe constitué d'un acide polylactique, d'un acide polyglycolique, d'un polyorthoester, d'un polyanhydride, du collagène et des copolymères, mélanges et combinaisons de ceux-ci.

18. Structure de feuille de matrice selon la revendication 15, dans laquelle la matière est une matière non dégradable, ou non absorbable, choisie dans le groupe constitué du polypropylène, du polyéthylène téréphtalate et autres polyesters, du polytétrafluoroéthylène, de l'éthylène-acétate de vinyle, du nylon, de l'acier inoxydable et des combinaisons de ceux-ci.

19. Structure de feuille de matrice selon l'une quelconque des revendications 15 à 18, dans laquelle la matière de la matrice est revêtue avec un facteur d'attachement choisi dans le groupe constitué des constituants de membrane basilaire, de la gélose, de l'agarose, de la gélatine, de la gomme arabique, des collagènes, de la fibronectine, de la laminine, de l'acide hyaluronique, des glycosaminoglucanes, des peptides d'attachement et des mélanges de ceux-ci.

20. Structure de feuille de matrice selon l'une quelconque des revendications 15 à 19, dans laquelle la matrice comprend en outre un composé choisi dans le groupe des composés angiogéniques, des substances nutritives, des facteurs de croissance, des inducteurs de différentiation ou de dé-différentiation, des produits de sécrétion, des immunomodulateurs, des inhibiteurs d'inflammation, des facteurs de régression, des composés biologiquement actifs qui accroissent ou permettent le développement interne du réseau lymphatique ou des fibres nerveuses, et des mélanges de ceux-ci.

21. Structure de feuille de matrice selon l'une quelconque des revendications 15 à 20, dans laquelle les cellules sont choisies dans le groupe constitué des hépatocytes, des cellules pancréatiques, des cellules surrénales, des cellules lymphoïdes, des cellules du système nerveux, des fibroblastes, des cellules endothéliales, des cellules spléniques et des cellules du système génito-urinaire.

22. Structure de feuille de matrice selon l'une quelconque des revendications 15 à 17 ou l'une quelconque des revendications 19 à 21 lorsqu'elles ne dépendent pas de la revendication 18, formée en revêtant un treillis fibreux biocompatible avec une seconde solution de polymère biocompatible, biodégradable, dans laquelle la matière du treillis fibreux n'est pas soluble dans la seconde solution de polymère.

23. Structure de feuille de matrice selon la revendication 22, dans laquelle le treillis fibreux est formé d'un acide polyglycolique et le second polymère est choisi dans le groupe d'un acide polylactique et des copolymères poly(acide lactique-acide glycolique.

24. Structure de feuille de matrice selon la revendication 22 ou 23, dans laquelle le second polymère forme des surfaces plates entre les fibres formant le treillis fibreux.
